# EUROPEAN PATENT APPLICATION

(11) **EP 4 653 441 A1**
(43) Date of publication of application: **26.11.2025**
(21) Application number: 24744339.3
(22) Date of filing: 18.01.2024
(51) Int. Cl.: C07D 487/04, A61P 25/24, A61K 31/407

(54) **SALT OF ANTIDEPRESSANT COMPOUND, AND PREPARATION METHOD THEREFOR, PHARMACEUTICAL COMPOSITION CONTAINING SAME AND USE THEREOF**

(30) Priority: 18.01.2023 CN 202310066877
(71) Applicant: Vigonvita Life Sciences Co., Ltd., Suzhou, Jiangsu 215123 (CN); Shanghai Institute of Materia Medica, Chinese Academy of Sciences, Shanghai 201203 (CN)
(72) Inventor: TIAN, Guanghui, Suzhou, Jiangsu 215123 (CN); CHENG, Yong, Suzhou, Jiangsu 215123 (CN); ZHANG, Yan, Shanghai 201203 (CN); YANG, Feipu, Shanghai 201203 (CN); HE, Yang, Shanghai 201203 (CN); WU, Chunhui, Suzhou, Jiangsu 215123 (CN)
(74) Representative: Grünecker Patent- und Rechtsanwälte PartG mbB
(86) International application number: PCT/CN2024/072942
(87) International publication number: WO 2024/153165

(57) **Abstract**

The present invention relates to a salt of a compound of formula (I), and a method for preparing the salt, a pharmaceutical composition containing the salt and the use thereof. The salt-forming acid of the salt of the compound of formula (I) is selected from a plurality of acids such as hydrochloric acid, maleic acid, hydrogen bromide, phosphoric acid and sulfuric acid. The salt of the compound of formula (I) has the advantages of a good stability, high solubility, low hygroscopicity, etc, and can be used for preparing a drug for treating and/or relieving depression.

## Description

### Cross-reference to related applications

This application claims priority to Chinese Patent Application No. 202310066877.X, filed on January 18, 2023, entitled "Salt of Antidepressant Compound, and Preparation Method Therefor, Pharmaceutical Composition Comprising the Same, and Use Thereof", the entire contents of which are incorporated herein by reference in their entirety to the same extent as if fully set forth herein.

### Technical Field

The present invention belongs to the technical field of medicine, and particularly relates to an acid addition salt of an antidepressant compound 2-methyl-5-(naphthalen-2-yl)octahydropyrrolo[3,4-c]pyrrole, a preparation method of the salt, a pharmaceutical composition comprising the salt, and a use of the salt or the pharmaceutical composition in the preparation of a medicine for treating and/or relieving depression.

### Background Art

Depression has become the fourth largest disease in the world, and according to "Depression and Other Common Mental Disorders Global Health Estimates" published by WHO in 2017, the total number of people suffering from depression in the world is currently 322 million, and nearly half of these people live in Southeast Asia and Western Pacific regions (including India and China). In China, the lifetime prevalence of depression is 6.9%, and the 12-month prevalence is 3.6%. With the increase of social rhythm, people experience increasing pressure in life, spirit and society, and the incidence of depression is increasing. Depression has the characteristics of high incidence, high recurrence and high disability, and has significant emotional, cognitive and somatic symptoms during the onset period, which brings serious troubles to the learning, work and life of patients and brings heavy economic burdens to society and families.

The existing antidepressants have various side effects such as nausea, vomiting, dyspepsia, diarrhea, headache, insomnia, drowsiness and anxiety, especially gastrointestinal side effects. At the same time, antidepressants generally need to be continuously administered for 2-4 weeks to be effective, and thus have a high discontinuation rate. Further, in addition to emotional and somatic symptoms, cognitive symptoms are also one of the main clinical manifestations of depression. Cognitive symptoms are mostly characterized by slow thinking, inattention, distraction, and diminished information processing ability. The symptom group is not only a common symptom manifestation in the acute phase of depression, but also an important residual symptom affecting function, and if not fully relieved, the patient's work, chores and academic ability cannot be restored to the past level, thereby reducing the quality of life. Residual cognitive symptoms are also one of the influencing factors of depression recurrence. Therefore, it is of great significance to reduce the gastrointestinal side effects of antidepressants, increase the patient's medication compliance, thereby reducing the discontinuation rate, and relieve the cognitive symptoms that are liable to be ignored clinically to promote the overall recovery of the function of depression patients.

WO2020239073A1 discloses a class of compounds that can inhibit the reuptake of endogenous monoamines, such as dopamine, serotonin, and norepinephrine (e.g., from the synaptic cleft), and/or modulate the 5-HT3 receptor by modulating one or more monoamine transporters, the general structural formula of which is shown below: 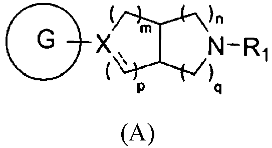 wherein, 2-methyl-5-(naphthalen-2-yl)octahydropyrrolo[3,4-c]pyrrole of the following formula (I) is specifically disclosed:

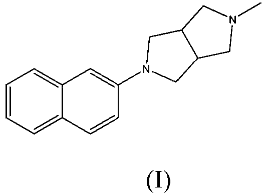

The compound showed a serotonin reuptake inhibition concentration lower than 10 nM (IC₅₀), and had a high affinity for 5-HT3 receptor. The compound not only has strong activity, low pharmacodynamic dose, low toxic and side effects, but also has therapeutic effects on diseases in the central nervous system field, and especially has a good druggable development prospect for diseases such as depression. However, the solubility of the compound is low, which limits its application in the pharmaceutical process to some extent.

### Summary of the Invention

### Problems to be Solved by the Invention

An object of the present invention is to provide an acid addition salt of 2-methyl-5-(naphthalen-2-yl)octahydropyrrolo[3,4-c]pyrrole with stable properties, high solubility and good druggability, a crystal form of the salt, and a pharmaceutical composition comprising the salt or the crystal form, and to provide a corresponding preparation method and a pharmaceutical use.

### Solutions to Solve the Problems

In a first aspect, the present invention provides a salt of the compound of formula (I), wherein the acid for forming the salt is selected from maleic acid, succinic acid, citric acid, tartaric acid, fumaric acid, formic acid, acetic acid, propionic acid, malonic acid, oxalic acid, benzoic acid, phthalic acid, methanesulfonic acid, ethanesulfonic acid, benzenesulfonic acid, toluenesulfonic acid, naphthalenesulfonic acid, 1,5-naphthalenedisulfonic acid, camphoric acid, camphorsulfonic acid, salicylic acid, acetylsalicylic acid, aspartic acid, glutamic acid, lactic acid, gluconic acid, ascorbic acid, gallic acid, mandelic acid, malic acid, citric acid, fumaric acid, succinic acid, sorbic acid, trifluoroacetic acid, taurine, homotaurine, 2-hydroxyethanesulfonic acid, cinnamic acid, mucic acid, hydrochloric acid, hydrogen bromide, hydrogen iodide, sulfuric acid, nitric acid, phosphoric acid, perchloric acid or a combination thereof,

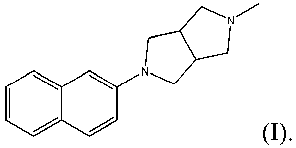

In a specific embodiment, the acid for forming the salt is selected from hydrochloric acid, maleic acid, sulfuric acid, hydrogen bromide, phosphoric acid, methanesulfonic acid, citric acid and fumaric acid;
preferably, the acid for forming the salt is selected from hydrochloric acid, maleic acid, hydrogen bromide, phosphoric acid and sulfuric acid;
more preferably, the acid for forming the salt is hydrochloric acid or maleic acid.

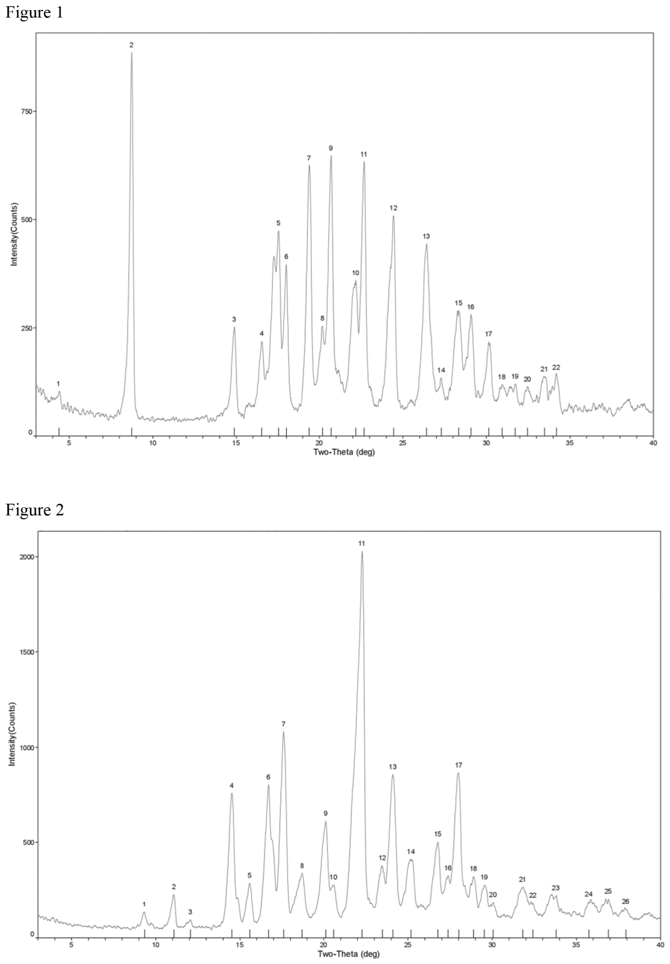

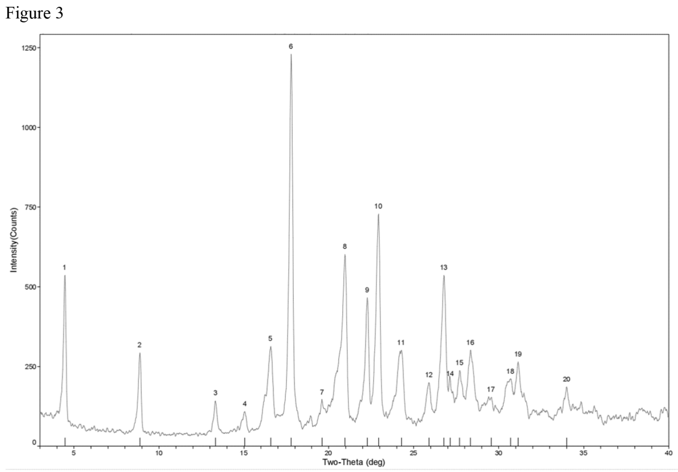

In one embodiment, the salt of the compound of formula (I) according to the present invention is a crystal form, comprising: crystal form A of the hydrochloride of the compound of formula (I), or crystal form B of the maleate of the compound of formula (I), and crystal form C of the hydrobromide of the compound of formula (I);
wherein the XRPD pattern of the crystal form A of the hydrochloride of the compound of formula (I) has characteristic peaks at the following 2θ diffraction angles: 8.74, 17.53, 17.99, 19.38, 20.70, 22.68, 24.44, 26.42 ± 0.20°; particularly, the XRPD pattern of the crystal form A has characteristic peaks at the following 2θ diffraction angles: 8.74, 14.88, 17.53, 17.99, 19.38, 20.70, 22.18, 22.68, 24.44, 26.42 ± 0.20°; more particularly, the XRPD pattern of the crystal form A has characteristic peaks at the following 2θ diffraction angles: 4.38, 8.74, 14.88, 16.52, 17.53, 17.99, 19.38, 20.17, 20.70, 22.18, 22.68, 24.44, 26.42, 27.29, 28.34, 29.08, 30.14, 30.94, 31.72, 32.46, 33.48, 34.19 ± 0.20°; more preferably, the XRPD pattern of the crystal form A is as shown in FIG. 1;
the XRPD pattern of the crystal form B of the maleate of the compound of Formula (I) has characteristic peaks at the following 2θ diffraction angles: 11.07, 14.52, 16.70, 17.60, 20.12, 22.28, 24.10, 28.01 ± 0.20°; particularly, the XRPD pattern of the crystal form B has characteristic peaks at the following 2θ diffraction angles: 11.07, 14.52, 16.70, 17.60, 20.12, 22.28, 24.10, 25.18, 26.77, 28.01 ± 0.20°; more particularly, the XRPD pattern of the crystal form B has characteristic peaks at the following 2θ diffraction angles: 9.33, 11.07, 12.06, 14.52, 15.58, 16.70, 17.60, 18.70, 20.12, 20.58, 22.28, 23.47, 24.10, 25.18, 26.77, 27.38, 28.01, 28.90, 29.54, 30.06, 31.82, 32.42, 33.80, 35.74, 36.90, 37.93 ± 0.20°; more preferably, the XRPD pattern of the crystal form B is as shown in FIG. 2;
the XRPD pattern of the crystal form C of the hydrobromide of the compound of formula (I) has characteristic peaks at the following 2θ diffraction angles: 4.47, 8.88, 16.58, 17.78, 20.96, 22.27, 22.94, 26.78 ± 0.20°; particularly, the XRPD pattern of the crystal form C has characteristic peaks at the following 2θ diffraction angles: 4.47, 8.88, 16.58, 17.78, 20.96, 22.27, 22.94, 24.28, 26.78, 28.34 ± 0.20°; more particularly, the XRPD pattern of the crystal form C has characteristic peaks at the following 2θ diffraction angles: 4.47, 8.88, 13.32, 15.04, 16.58, 17.78, 19.61, 20.96, 22.27, 22.94, 24.28, 25.90, 26.78, 27.15, 27.70, 28.34, 29.56, 30.70, 31.14, 34.00 ± 0.20°; more preferably, the XRPD pattern of the crystal form C is as shown in FIG. 3.

In a second aspect, the present invention provides a process for preparing the salt of the compound of formula (I) according to the first aspect of the present invention, comprising the following steps:
1) dissolving the compound of formula (I) in a solvent, heating to 40-60° C (e.g., 50 °C, 55 °C), stirring and dissolving to obtain a solution A, adding an acid for forming the salt to the solution A, stirring, precipitating, and continuing to stir for 0.5-4 h;
2) cooling the mixture in step 1) to 0-30 °C (e.g., 5 °C, 10 °C), and continuing to stir for 0.5-4 h;
3) filtering, drying the filter cake under normal pressure at 30-60 °C (e.g., 50 °C, 55 °C) to obtain the salt of the compound of formula (I).

In a specific embodiment, in step 1), the solvent is selected from acetone, an alcohol solvent (such as methanol, ethanol, isopropanol, etc.), acetonitrile, isopropyl acetate, ethyl acetate, dichloromethane, chloroform, toluene or a combination thereof; preferably, the solvent is selected from acetone, anhydrous ethanol, isopropanol, methanol, acetonitrile, isopropyl acetate, ethyl acetate or a combination thereof.

In a specific embodiment, in step 1), the ratio of the compound of formula (I) to the solvent is 1 g: 2-25 mL, preferably 1 g: 3-15 mL.

In a specific embodiment, in step 1), the molar ratio of the compound of formula (I) to the acid is 1:0.9-1.2.

In a specific embodiment, in step 1), the stirring is continued for 0.5-2 hours.

In a specific embodiment, in step 2), the stirring is continued for 0.5-2 hours.

In a specific embodiment, the salt of the compound of formula (I) prepared according to the preparation process of the present invention is a crystal form, for example, crystal form A of the hydrochloride of the compound of formula (I), crystal form B of the maleate of the compound of formula (I), and the crystal form C of the hydrobromide of the compound of formula (I) as described above.

In a third aspect, the present invention provides a pharmaceutical composition comprising a therapeutically effective amount of the salt of the compound of formula (I) of the first aspect; and optionally a pharmaceutically acceptable adjuvant.

In a specific embodiment, the pharmaceutical composition is an oral formulation or a non-oral formulation. Preferably, the oral preparation is selected from tablets, capsules, granules, powders and syrups; and the non-oral preparation is selected from injections, powder injections, sprays and suppositories.

In a fourth aspect, the present invention provides a use of the salt of the compound of formula (I) according to the first aspect or the pharmaceutical composition according to the third aspect of the present invention in the preparation of a medicine for treating and/or alleviating depression.

### Beneficial Effects

In the present invention, the acid addition salt of the compound (2-methyl-5-(naphthalen-2-yl)octahydropyrrolo[3,4-c]pyrrole) having antidepressant effect and the crystal thereof are synthesized, isolated, and studied in related physicochemical properties, and the salt (and its corresponding crystal form) having the advantages of good solid properties, good stability, high solubility, low hygroscopicity and the like is found. It can be used to prepare medicines for treating and/or relieving depression.

### Brief Description of Drawings

FIG. 1 shows the XRPD pattern of the hydrochloride crystal form A of the compound of formula (I) prepared in Example 1.
FIG. 2 shows the XRPD pattern of the maleate crystal form B of the compound of formula (I) prepared in Example 7.
FIG. 3 shows the XRPD pattern of the hydrobromide crystal form C of the compound of formula (I) prepared in Example 2.

### Detailed Description

### [Definition of Terms]

### Pharmaceutical Compositions

Unless otherwise specified, the "pharmaceutical composition" described in the present invention comprises at least one salt of the compound of the present invention (including the crystal form of the salt), and optionally pharmaceutically acceptable adjuvants.

Unless otherwise specified, the "adjuvant" described in the present invention includes (but is not limited to) excipients, binders, lubricants, disintegrants, colorants, flavoring agents, olfactory agents, emulsifiers, surfactants, co-solvents, suspending agents, isotonic agents, buffers, preservatives, antioxidants, stabilizers, absorption enhancers and the like commonly used in the pharmaceutical field, and the above adjuvants can also be used after being appropriately combined as required.

In an oral pharmaceutical composition, the salt of the compound of the present invention is mixed with at least one pharmaceutically acceptable adjuvant to prepare a formulation with an active pharmaceutical ingredient (API) of 10-2000 mg per unit dose. For example, when the oral pharmaceutical composition is a tablet, the API is mixed with at least one pharmaceutical adjuvant (e.g., starch, lactose, magnesium stearate, etc.) and compressed, and the tablet may be further coated with a sugar coating or other suitable substances, or treated, so that the tablet has a sustained-release or controlled-release effect. As another example, when the oral pharmaceutical composition is a capsule, the API is mixed with at least one diluent (e.g., starch) and optionally granulated, pelletized, and the resulting mixture is loaded into a capsule shell.

### Therapeutically Effective Amount

Unless otherwise specified, the "therapeutically effective amount" in the present invention refers to the amount of the salt of the compound of the present invention and/or the crystal form of the salt that can cause a biological or medical response or ameliorate symptoms, slow or delay the progression of disease or prevent disease in an individual. A "therapeutically effective amount" may be determined by the participating physician or veterinarian practitioner and will vary with the salt of the compound and/or the crystal form of the salt, the disease state being treated, the severity of the disease being treated, the age and associated health of the individual, the route and form of administration, the judgment of the attending physician or veterinarian practitioner, and the like. Taking adults as an example, the drug can be administered 1 to 7 times every 1 to 7 days according to the symptoms, with an administration amount of about 0.01 to 1000 mg, and the administration mode is not limited.

The salt of the compound or the pharmaceutical composition of the present invention can be used to treat and/or relieve symptoms associated with depression, and the salt of the compound or the pharmaceutical composition of the present invention can also be used to prepare medicines for treating and/or relieving depression.

The present invention will be further described below with reference to specific examples. It should be understood that these examples are only used to illustrate the present invention, and are not used to limit the scope of the present invention. The experimental methods without specific conditions in the following examples are generally performed under conventional conditions or according to the conditions recommended by the manufacturer. Unless otherwise stated, the percentages and parts in the present invention are calculated by weight.

### Description of Reagents and Consumables

In the examples of the present disclosure, the reagents such as anhydrous ethanol involved in the preparation method are all analytically pure and provided by Sinopharm Chemical Reagent Co., Ltd., unless otherwise specified, the reagents used are not specially treated. 2-methyl-5-(naphthalen-2-yl)octahydropyrrolo[3,4-c]pyrrole was prepared with reference to Example 2 in CN202010474768.8, and the purity was greater than 99%. The trifluoroacetic acid and acetonitrile involved in the high performance liquid chromatography experiment are chromatographic pure.

### General Test Methods

1. X-ray Powder Diffraction (XRPD) Test Method:
Instrument: Bruker D8 advance X-ray polycrystalline diffractometer; Target: Cu-Kα (40 kV, 40 mA); Sample-to-detector distance: 30 cm; Scanning type: two-axis linkage; Scanning step width: 0.02°; Scanning range: 3°~40°; Scanning step: 0.1 s.
In general, diffraction angles (2θ values) in XRPD may produce errors within a range of ± 0.2°, and thus numerical values relating to diffraction angles in the present invention should be understood to also encompass numerical values within a range of about ± 0.2°. Therefore, the present invention not only covers the crystal form that completely matches the characteristic signal peak in the specific XRPD pattern, but also covers the crystal form that has an error of about ± 0.2° with the characteristic signal peak in the specific XRPD pattern.

2. Solubility Test Method:
About 20 mg of the test sample was taken and placed in a 1.5 ml sample tube, added with 1 ml of water, then placed in a shaker, shaken at 37°C for 30 minutes, the sample tube was taken and centrifuged in a centrifuge for 2 minutes, and the supernatant was taken for HPLC analysis. The solubility of the test sample in water ((37°C) was calculated by an external standard method.
HPLC Chromatography Conditions:
Column: Waters Xselect CSH Phenyl-Hexyl 4.6 × 150 mm 3.5 µm
Flow rate: 1.0 mL/min
Detection wavelength: 230 nm
Column oven: 30° C
Injection volume: 10 µL
Diluent: acetonitrile-water = 1 : 1 (v/v)
Injection volume: 5 ul
Mobile Phase:
Mobile phase A: 0.05% trifluoroacetic acid in water
Mobile phase B: 0.05% trifluoroacetic acid in acetonitrile
The gradient elution procedure is shown in Table 1:

**Table 1 HPLC Gradient Elution Procedure**

| Time (min) | A (%) | B (%) |
|---|---|---|
| 0 | 90 | 10 |
| 20 | 30 | 70 |
| 23 | 20 | 80 |
| 23.1 | 90 | 10 |
| 30 | 90 | 10 |

3. Hygroscopicity Measurement Method:
The test sample was placed for 24 hours at 25°C and 92.5% RH, and then the weight gain by moisture absorption was measured.
4. Stability Test Method:
The test sample was placed in a suitable clean container for 14 days under a high temperature (60°C), or a high humidity (40°C, a relative humidity of 75%) condition, and it was sampled on day 0 and day 14 to detect the purity and impurities of the test sample.

### Example 1: Preparation of the hydrochloride of the compound of formula (I)

Acetone (5 ml) was added to the compound of formula (I) (0.5 g, 1.98 mmol, 1 eq), and the mixture was heated to 40 °C with stirring to dissolve. Concentrated hydrochloric acid (227 mg, 2.18 mmol, 1.1 eq) was added, and the mixture was stirred at 40°C for half an hour. Solid was precipitated, and the stirring was continued at 40°C for 1 hour. The mixture was cooled to 5°C and stirred for 1 hour, then filtered. The filter cake was collected, and dried at 55±5°C under normal pressure to obtain the hydrochloride of the compound of formula (I) (0.45 g, molar yield 78.9%, purity 99.72%)..

### Example 2: Preparation of the hydrobromide of the compound of formula (I)

Anhydrous ethanol (5 ml) was added to the compound of formula (I) (0.5 g, 1.98 mmol, 1 eq), and the mixture was heated to 50 °C with stirring to dissolve. Aqueous solution of hydrogen bromide (440 mg, 2.18 mmol, 1.1 eq) was added, and the mixture was stirred at 50°C for half an hour. Solid was precipitated, and the stirring was continued at 50°C for 1 hour. The mixture was cooled to 5°C and stirred for 1 hour, then filtered. The filter cake was collected, and dried at 55±5°C under normal pressure to obtain the hydrobromide of the compound of formula (I) (0.6 g, molar yield 91.1%, purity 99.60%).

### Example 3: Preparation of the mesylate of the compound of formula (I)

Anhydrous ethanol (5 ml) was added to the compound of formula (I) (0.5 g, 1.98 mmol, 1 eq), and the mixture was heated to 55 °C with stirring to dissolve. Methanesulfonic acid (210 mg, 2.18 mmol, 1.1 eq) was added, and the mixture was stirred at 55°C for half an hour. A large amount of solid was precipitated, and the stirring was continued at 55°C for 1 hour. The mixture was cooled to 10°C and stirred for 1 hour, then filtered. The filter cake was collected, and dried at 55±5°C under normal pressure to obtain the mesylate of the compound of formula (I) (0.23, molar yield 33.4%, purity 99.93%).

### Example 4: Preparation of the sulfate of the compound of formula (I)

Anhydrous ethanol (5 ml) was added to the compound of formula (I) (0.5 g, 1.98 mmol, 1 eq), and the mixture was heated to 50 °C with stirring to dissolve. Concentrated sulfuric acid (194 mg, 2.18 mmol, 1.1 eq) was added, and the mixture was stirred at 50°C for half an hour. A large amount of solid was precipitated, and the stirring was continued at 50°C for 1 hour. The mixture was cooled to 10°C and stirred for 1 hour, then filtered. The filter cake was collected and dried at 55±5°C under normal pressure to obtain the sulfate of the compound of formula (I) (0.64 g, molar yield 92%, purity 99.57%).

### Example 5: Preparation of the phosphate of the compound of formula (I)

Anhydrous ethanol (5 ml) was added to the compound of formula (I) (0.5 g, 1.98 mmol, 1 eq), and the mixture was heated to 55 °C with stirring to dissolve. Phosphoric acid (214 mg, 2.18 mmol, 1.1 eq) was added, and the mixture was stirred at 55°C for half an hour. Solid was precipitated, and the stirring was continued at 55°C for 1 hour. The mixture was cooled to 10°C and stirred for 1 hour, and then filtered. The filter cake was collected, and dried at 55±5°C under normal pressure to obtain the phosphate of the compound of formula (I) (0.45 g, molar yield 78.9%, purity 99.72%).

### Example 6: Preparation of the citrate of the compound of formula (I)

Anhydrous ethanol (5 ml) was added to the compound of formula (I) (0.5 g, 1.98 mmol, 1 eq), and the mixture was heated to 50°C with stirring to dissolve. Monohydrated citric acid (456 mg, 2.18 mmol, 1.1 eq) was added, and the mixture was stirred at 50°C for half an hour. Then solid was precipitated, and the stirring was continued at 50°C for 1 hour. The mixture was cooled to 10°C and stirred for 1 hour, then filtered. The filter cake was collected and dried at 55±5°C under normal pressure to obtain the citrate of the compound of formula (I) (0.89 g, molar yield 97.9%, purity 99.62%).

### Example 7: Preparation of the maleate of the compound of formula (I)

Anhydrous ethanol (1.5 ml) was added to the compound of formula (I) (0.5 g, 1.98 mmol, 1 eq), and the mixture was heated to 55°C with stirring to dissolve. Maleic acid (275 mg, 2.37 mmol, 1.2 eq) was added, and the mixture was stirred at 55°C for half an hour. Solid was precipitated, and the stirring was continued at 55°C for 1 hour. The mixture was cooled to 10°C and stirred for 1 hour, then filtered. The filter cake was collected and dried at 55±5°C under normal pressure to obtain the maleate of the compound of formula (I) (0.6 g, molar yield 87%, purity 99.21%).

### Example 8: Preparation of the hydrochloride of the compound of formula (I)

Anhydrous ethanol (7 ml) was added to the compound of formula (I) (0.5 g, 1.98 mmol, 1 eq), and the mixture was heated to 45°C with stirring to dissolve. Concentrated hydrochloric acid (210 mg, 1.98 mmol, 1 eq) was added, and the mixture was stirred at 45°C for half an hour. Solid was precipitated, and the stirring was continued at 45°C for 1 hour. The mixture was cooled to 5°C and stirred for 1 hour, then filtered. The filter cake was collected, and dried at 50°C under normal pressure to obtain the hydrochloride of the compound of formula (I) (0.42 g, molar yield 74%, purity 99.97%).

### Example 9: Preparation of the hydrochloride of the compound of formula (I)

Isopropanol (5 ml) was added to the compound of the formula (I) (0.5 g, 1.98 mmol, 1 eq), and the mixture was heated to 50°C with stirring to dissolve. Concentrated hydrochloric acid (227 mg, 2.18 mmol, 1.1 eq) was added, and the mixture was stirred at 50°C for half an hour. Solid was precipitated, and the stirring was continued at 50°C for 1 hour. The mixture was cooled to 10°C and stirred for 1 hour, then filtered. The filter cake was collected and dried at 55±5°C under normal pressure to obtain the hydrochloride of the compound of formula (I) (0.45 g, molar yield 78%, purity 99.73%).

### Example 10: Preparation of the hydrochloride of the compound of formula (I)

Acetonitrile (5 ml) was added to the compound of formula (I) (0.5 g, 1.98 mmol, 1 eq), and the mixture was heated to 55°C with stirring to dissolve. Concentrated hydrochloric acid (227 mg, 2.18 mmol, 1.1 eq) was added, and the mixture was stirred at 55°C for half an hour. Solid was precipitated, and the stirring was continued at 55°C for 1 hour. The mixture was cooled to 15°C and stirred for 1 hour, then filtered. The filter cake was collected, and dried at normal pressure 55±5°C to obtain the hydrochloride of the compound of formula (I) (0.43 g, molar yield 74.5%, purity 99.95%).

### Example 11: Preparation of the hydrochloride of the compound of formula (I)

Isopropyl acetate (10 ml) and anhydrous ethanol (1.5 ml) were added to the compound of formula (I) (0.5 g, 1.98 mmol, 1 eq), and the mixture was heated to 50°C with stirring to dissolve. Concentrated hydrochloric acid (227 mg, 2.18 mmol, 1.1 eq) was added, and the mixture was stirred at 50°C for half an hour. Solid was precipitated, and the stirring was continued at 50°C for 1 hour. The mixture was cooled to 10°C and stirred for 1 hour, then filtered. The filter cake was collected and dried at 55±5°C under normal pressure to obtain the hydrochloride of the compound of formula (I) (0.56 g, molar yield 98.7%, purity 99.5%).

### Example 12: Preparation of the hydrochloride of the compound of formula (I)

Ethyl acetate (10 ml) and anhydrous ethanol (1.5 ml) were added to the compound of formula (I) (0.5 g, 1.98 mmol, 1 eq), and the mixture was heated to 55°C with stirring to dissolve. Concentrated hydrochloric acid (227 mg, 2.18 mmol, 1.1 eq) was added, and the mixture was stirred at 55°C for half an hour. A large amount of solid was precipitated, and the stirring was continued at 55°C for 1 hour. The mixture was cooled to 15°C and stirred for 1 hour, then filtered. The filter cake was collected, and dried at 55°C under normal pressure to obtain the hydrochloride of the compound of formula (I) (0.55 g, molar yield 98.2%, purity 99.4%).

The salts of the compound of formula (I) prepared in the above examples were subjected to X-ray powder diffraction (XRPD), hygroscopicity, solubility and stability tests according to the above methods. The results are as follows:

### X-ray Powder Diffraction (XRPD) Test Results:

The hydrochloride of the compound of formula (I) prepared in Example 1 is in a crystal form, corresponding to crystal form A, and the X-ray powder diffraction information is shown in Table 2 and FIG. 1. The hydrochloride of the compound of formula (I) prepared in Example 8-12 has similar XRPD pattern to that in FIG. 1.

**Table 2 XRPD data of crystal form A of the hydrochloride of the compound of formula (I)**

| Peak Number | Diffraction Angle (2θ, °) | Intensity (I/I₀, %) | Peak Number | Diffraction Angle (2θ, °) | Intensity (I/I₀, %) |
|---|---|---|---|---|---|
| 1 | 4.38 | 3.8 | 12 | 24.44 | 52.5 |
| 2 | 8.74 | 100 | 13 | 26.42 | 42.3 |
| 3 | 14.88 | 24.3 | 14 | 27.29 | 4.3 |
| 4 | 16.52 | 19.7 | 15 | 28.34 | 23.3 |
| 5 | 17.53 | 48.7 | 16 | 29.08 | 18.2 |
| 6 | 17.99 | 39.7 | 17 | 30.14 | 15.5 |
| 7 | 19.38 | 62.2 | 18 | 30.94 | 4.4 |
| 8 | 20.17 | 15.1 | 19 | 31.72 | 2.81 |
| 9 | 20.70 | 63.6 | 20 | 32.46 | 2.75 |
| 10 | 22.18 | 31.1 | 21 | 33.48 | 2.67 |
| 11 | 22.68 | 63.7 | 22 | 34.19 | 2.61 |

The maleate of the compound of formula (I) prepared in Example 7 is in crystal form, corresponding to crystal form B, and its X-ray powder diffraction information is shown in Table 3 and FIG. 2.

**Table 3 the XRPD data of the crystal form B of the maleate of the compound of formula (I)**

| Peak Number | Diffraction Angle (2θ, °) | Intensity (I/I₀, %) | Peak Number | Diffraction Angle (2θ, °) | Intensity (I/I₀, %) |
|---|---|---|---|---|---|
| 1 | 9.33 | 4.7 | 14 | 25.18 | 13.2 |
| 2 | 11.07 | 26 | 15 | 26.77 | 15.4 |
| 3 | 12.06 | 2.1 | 16 | 27.38 | 4.6 |
| 4 | 14.52 | 36.5 | 17 | 28.01 | 34.2 |
| 5 | 15.58 | 10.8 | 18 | 28.90 | 6.9 |
| 6 | 16.70 | 38.6 | 19 | 29.54 | 6.6 |
| 7 | 17.60 | 47.3 | 20 | 30.06 | 3.1 |
| 8 | 18.70 | 11.8 | 21 | 31.82 | 8.2 |
| 9 | 20.12 | 27.0 | 22 | 32.42 | 2.7 |
| 10 | 20.58 | 8.1 | 23 | 33.80 | 5.6 |
| 11 | 22.28 | 100 | 24 | 35.74 | 3.6 |
| 12 | 23.47 | 11.2 | 25 | 36.90 | 4.1 |
| 13 | 24.10 | 36.4 | 26 | 37.93 | 2.4 |

The hydrobromide of the compound of formula (I) prepared in Example 2 is in crystal form, corresponding to crystal form C, and its X-ray powder diffraction information is shown in Table 4 and FIG. 3.

**Table 4 the XRPD data of the crystal form C of the hydrobromide of the compound of formula (I)**

| Peak Number | Diffraction Angle (2θ, °) | Intensity (I/I₀, %) | Peak Number | Diffraction Angle (2θ, °) | Intensity (I/I₀, %) |
|---|---|---|---|---|---|
| 1 | 4.47 | 39.7 | 11 | 24.28 | 18.9 |
| 2 | 8.88 | 22.1 | 12 | 25.90 | 8.8 |
| 3 | 13.32 | 8.9 | 13 | 26.78 | 35.4 |
| 4 | 15.04 | 5.4 | 14 | 27.15 | 5.5 |
| 5 | 16.58 | 19.7 | 15 | 27.70 | 7.8 |
| 6 | 17.78 | 100 | 16 | 28.34 | 15.2 |
| 7 | 19.61 | 4.1 | 17 | 29.56 | 3.7 |
| 8 | 20.96 | 43.9 | 18 | 30.70 | 9.1 |
| 9 | 22.27 | 32.8 | 19 | 31.14 | 14.2 |
| 10 | 22.94 | 52.6 | 20 | 34.00 | 7.9 |

### Hygroscopicity Test Results

The results are shown in Table 5.

**Table 5 Hygroscopicity of salts of the compound of formula (I)**

| Examples | 1 | 2 | 3 | 4 | 5 | 6 | 7 |
|---|---|---|---|---|---|---|---|
| Types of the Salt | Hydrochlorid e | Hydrobromid e | Mesylat e | Sulfat e | Phosphat e | Citrate | Maleat e |
| weight gain by moisture | **1.6%** | **0.74%** | To be liquid | 6.74% | **1.21%** | **1.26 %** | **0.64%** |
| absorptio n | | | | | | | |

Due to the structural characteristics, the salts of the compound of formula (I) prepared in Example 1-7 will have a certain hygroscopicity, and it is found unexpectedly by experiments that the hydrochloride, hydrobromide, maleate, phosphate and citrate have lower hygroscopicity than other salts, which are more conducive to storage and preparation into a formulation.

### Solubility Test Results:

The results are shown in Table 6.

**Table 6 Solubility (mg/mL) of the salts of the compound of formula (I)**

| Examples | 1 | 2 | 4 | 5 | 7 | |
|---|---|---|---|---|---|---|
| Types of Salts | Hydrochloride | Hydrobromide | Sulfate | Phosphate | Maleate | basic compound of formula (I) |
| Solubility | **>100** | **11** | 3.5 | 4 | **15** | Almost Insoluble |

It can be seen from the data in Table 6 that, when the compound of formula (I) is formed as a salt, the solubility is significantly improved, especially hydrochloride, hydrobromide and maleate have significantly improved solubility as compared with other salts. The improvement of solubility significantly improves the druggability of the compound of formula (I), and solves the problem that a stable preparation cannot be obtained due to the solubility problem.

### Analysis of Stability Test Results:

**Table 7 Stability of the salt of the compound of formula (I)**

| Examples | Salt Type | Conditions/Time | Total Impurity (%) |
|---|---|---|---|
| 1 | Hydrochl oride | Day 0 | 0.13 |
| | | 60°C/14 days | 0.19 |
| | | 60°C/14 days (aluminum foil pouch) | 0.19 |
| | | 40°C/75% RH/14 days | 0.15 |
| 7 | Maleate | Day 0 | 0.14 |
| | | 60°C/14 days | 0.18 |
| | | 60°C/14 days | 0.20 |
| | | (aluminum foil pouch) | |
| | | 40°C/75% RH/14 days | 0.17 |
| 2 | Hydrobro mide | Day 0 | 0.35 |
| | | 60°C/14 days | 0.37 |
| | | 60°C/14 days (aluminum foil pouch) | 0.36 |
| | | 40°C/75% RH/14 days | 0.31 |
| 4 | Sulfate | Day 0 | 0.21 |
| | | 60°C/14 days | 0.22 |
| | | 60°C/14 days (aluminum foil pouch) | 0.24 |
| | | 40°C/75% RH/14 days | 0.44 |
| | Basic compoun d | Day 0 | 0.15 |
| | | 60°C/14 days | 0.23 |
| | | 60°C/14 days (aluminum foil pouch) | 0.31 |
| | | 40°C/75% RH/14 days | 0.41 |

As can be seen from the long-term stability data of Table 7, after being a salt, the stability of the compound of formula (I) is significantly improved, and the total impurity growth of the salt under high temperature and high humidity conditions is significantly lower than that of the basic compound. In addition, the stability of the hydrochloride and maleate thereof is superior to other salt types. The increased stability significantly improves the stability of the API in the preparation process and final preparation product.

The stability test was performed on the preparations of different salts of the compound of formula (I), and the preparation stability test method and results are as follows:

**Table 8 shows different salt formulations of the compound of formula (I).**

| Material Name | Unit Dose (mg) | | |
|---|---|---|---|
| | Formulation 1 | Formulation 2 | Formulation 3 |
| Salt type of the compound of Formula (I) | 1.14 (Hydrochloride) | 1.39 (Sulfate) | 1.46 (Maleate) |
| EDTA-2Na | 1.0 | 1.0 | 1.0 |
| Pregelatinized Starch | 97.36 | 97.11 | 97.04 |
| Sodium Stearyl Fumarate | 0.5 | 0.5 | 0.5 |
| Total | 100 | 100 | 100 |

| | | | |
|---|---|---|---|
| * Note: the amounts of different salts of the compound of formula (I) are converted according to the molecular weight of different salts, so as to contain the same amount of free base. The hydrochloride, sulfate, and maleate forms in Formulation 1-3 were prepared according to the methods of Examples 1 and 4 and 7, respectively. | | | |

In Table 8, the composition particles of different salts of the compound of formula (I) were prepared according to the following preparation steps:
(1) Preparation of raw materials and adjuvants: all raw materials and adjuvants were weighed according to the theoretical amount, the raw materials (different salt types of the compound of formula I) were sieved through a 60-mesh sieve, and the adjuvants (except for EDTA-2Na) were sieved through a 20-mesh sieve;
(2) Preparation of wetting agent: a formulated amount of EDTA- 2Na was added into an appropriate amount of purified water, and stirred until it was completely dissolved;
(3) wet granulation: the salt of the compound of formula (I) and pregelatinized starch were added into a wet granulator, and EDTA- 2Na aqueous solution was slowly added under stirring to prepare wet granules;
(4) Drying: the wet granules were added into a fluidized bed to dry until the drying weight loss is less than 7%;
(5) sizing: the dried particles were granulated by a grinding and granulate machine with a 1.5 mm sieve;
(6) Mixing: Sodium stearyl fumarate was added to the granulated dry granules and mixed for 1 min.

Preparation stability test: the samples prepared by the above formulation (double-aluminum packaging) were placed at a test condition 40±2°C/RH75 ± 5.0%, and it was sampled at 0 day, 1 month, 2 months and 3 months, respectively, to investigate the changes of related substances, and the results are shown in Table 9.

**Table 9 Preparation stability test results of different salts of the compound of formula (I)**

| Samples | Storage Time | The increased amount of the related substances over 0 day (40 ± 2°C/RH75 ± 5.0%, aluminum packaging sample) | |
|---|---|---|---|
| | | Maximum Degradation Impurities (%) | Total Impurities (%) |
| Formulation 1 | 1 month | 0.05 | 0.01 |
| | 2 months | 0.07 | 0.07 |
| | 3 months | 0.08 | 0.14 |
| Formulation 2 | 1 month | 0.05 | 0.04 |
| | 2 months | 0.07 | 0.18 |
| | 3 months | 0.08 | 0.34 |
| Formulation 3 | 1 month | 0.03 | 0.03 |
| | 2 months | 0.03 | 0.05 |
| | 3 months | 0.05 | 0.14 |

It can be seen from the preparation stability test results that the two preparation samples of maleate and hydrochloride of the compound of formula (I) have less impurity increase after being accelerated 3 months.

All documents mentioned in the present invention are incorporated herein by reference, as if each document is individually incorporated herein by reference. In addition, it should be understood that after reading the above contents of the present disclosure, those skilled in the art can make various adjustments or modifications to the present disclosure, and these equivalent forms also fall within the scope defined by the appended claims of the present disclosure.

## Claims

1. A salt of the compound of formula (I), wherein an acid for forming the salt is selected from maleic acid, succinic acid, citric acid, tartaric acid, fumaric acid, formic acid, acetic acid, propionic acid, malonic acid, oxalic acid, benzoic acid, phthalic acid, methanesulfonic acid, ethanesulfonic acid, benzenesulfonic acid, toluenesulfonic acid, naphthalenesulfonic acid, 1,5-naphthalenedisulfonic acid, camphoric acid, camphorsulfonic acid, salicylic acid, acetylsalicylic acid, aspartic acid, glutamic acid, lactic acid, gluconic acid, ascorbic acid, gallic acid, mandelic acid, malic acid, citric acid, fumaric acid, succinic acid, sorbic acid, trifluoroacetic acid, taurine, homotaurine, 2-hydroxyethanesulfonic acid, cinnamic acid, mucic acid, hydrochloric acid, hydrogen bromide, hydrogen iodide, sulfuric acid, nitric acid, phosphoric acid, and perchloric acid,

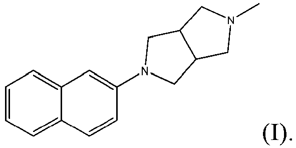

2. The salt of the compound of formula (I) according to claim 1, wherein the acid for forming the salt is selected from hydrochloric acid, maleic acid, sulfuric acid, hydrogen bromide, phosphoric acid, methanesulfonic acid, citric acid and fumaric acid;
preferably, the acid for forming the salt is selected from hydrochloric acid, maleic acid, hydrogen bromide, phosphoric acid and sulfuric acid;
more preferably, the acid for forming the salt is hydrochloric acid or maleic acid.

3. The salt of the compound of formula (I) according to claim 1, wherein the salt is hydrochloride of the compound of formula (I) as a crystal form A; or maleate of the compound of formula (I) as a crystal form B; or hydrobromide of the compound of formula (I) as a crystal form C;
wherein the XRPD pattern of the crystal form A of the hydrochloride of the compound of formula (I) has characteristic peaks at the following 2θ diffraction angles: 8.74, 17.53, 17.99, 19.38, 20.70, 22.68, 24.44, 26.42 ± 0.20°; preferably, the XRPD pattern of the crystal form A has characteristic peaks at the following 2θ diffraction angles: 8.74, 14.88, 17.53, 17.99, 19.38, 20.70, 22.18, 22.68, 24.44, 26.42 ± 0.20°; more preferably, the XRPD pattern of the crystal form A has characteristic peaks at the following 2θ diffraction angles: 4.38, 8.74, 14.88, 16.52, 17.53, 17.99, 19.38, 20.17, 20.70, 22.18, 22.68, 24.44, 26.42, 27.29, 28.34, 29.08, 30.14, 30.94, 31.72, 32.46, 33.48, 34.19 ± 0.20°; more preferably, the XRPD pattern of the crystal form A is as shown in FIG. 1;
the XRPD pattern of the crystal form B of the maleate of the compound of Formula (I) has characteristic peaks at the following 2θ diffraction angles: 11.07, 14.52, 16.70, 17.60, 20.12, 22.28, 24.10, 28.01 ± 0.20°; preferably, the XRPD pattern of the crystal form B has characteristic peaks at the following 2θ diffraction angles: 11.07, 14.52, 16.70, 17.60, 20.12, 22.28, 24.10, 25.18, 26.77, 28.01 ± 0.20°; more preferably, the XRPD pattern of the crystal form B has characteristic peaks at the following 2θ diffraction angles: 9.33, 11.07, 12.06, 14.52, 15.58, 16.70, 17.60, 18.70, 20.12, 20.58, 22.28, 23.47, 24.10, 25.18, 26.77, 27.38, 28.01, 28.90, 29.54, 30.06, 31.82, 32.42, 33.80, 35.74, 36.90, 37.93 ± 0.20°; more preferably, the XRPD pattern of the crystal form B is as shown in FIG. 2;
the XRPD pattern of the crystal form C of the hydrobromide of the compound of formula (I) has characteristic peaks at the following 2θ diffraction angles: 4.47, 8.88, 16.58, 17.78, 20.96, 22.27, 22.94, 26.78 ± 0.20°; preferably, the XRPD pattern of the crystal form C has characteristic peaks at the following 2θ diffraction angles: 4.47, 8.88, 16.58, 17.78, 20.96, 22.27, 22.94, 24.28, 26.78, 28.34 ± 0.20°; more preferably, the XRPD pattern of the crystal form C has characteristic peaks at the following 2θ diffraction angles: 4.47, 8.88, 13.32, 15.04, 16.58, 17.78, 19.61, 20.96, 22.27, 22.94, 24.28, 25.90, 26.78, 27.15, 27.70, 28.34, 29.56, 30.70, 31.14, 34.00 ± 0.20°; more preferably, the XRPD pattern of the crystal form C is as shown in FIG. 3.

4. A process for preparing the salt of the compound of formula (I) according to any one of claims 1 to 3, comprising the following steps:
1) dissolving the compound of formula (I) in a solvent, heating to 40-60° C, stirring and dissolving to obtain a solution A, adding an acid for forming the salt to the solution A, stirring, precipitating, and continuing to stir for 0.5-4 h;
2) cooling the mixture in step 1) to 0-30 °C, and continuing to stir for 0.5-4 h;
3) filtering, drying the filter cake under normal pressure at 30-60 °C to obtain the salt of the compound of formula (I).

5. The process according to claim 4, wherein in step 1), the solvent is selected from acetone, an alcohol solvent, acetonitrile, isopropyl acetate, ethyl acetate, dichloromethane, chloroform, toluene or a combination thereof; preferably, the solvent is selected from acetone, anhydrous ethanol, isopropanol, methanol, acetonitrile, isopropyl acetate, ethyl acetate or a combination thereof.

6. The process according to claim 4, wherein in step 1), the ratio of the compound of formula (I) to the solvent is 1 g: 2-25 mL, preferably 1 g: 3-15 mL; and/or
in step 1), the molar ratio of the compound of formula (I) to the acid is 1:0.9-1.2.

7. The process according to claim 4, wherein in step 1), the stirring is continued for 0.5-2 hours; and/or
in step 2), the stirring is continued for 0.5-2 hours.

8. A pharmaceutical composition comprising a therapeutically effective amount of the salt of the compound of formula (I) according to any one of claims 1 to 3; and optionally a pharmaceutically acceptable adjuvant.

9. The pharmaceutical composition according to claim 8, wherein the pharmaceutical composition is an oral formulation or a non-oral formulation;
preferably, the oral formulation is selected from tablets, capsules, granules, powders and syrups; and the non-oral formulation is selected from injections, powder injections, sprays and suppositories.

10. A use of the salt of the compound of formula (I) according to any one of claims 1 to 3 or a pharmaceutical composition according to claim 8 in the preparation of a medicine for treating and/or alleviating depression.
